# EUROPÄISCHE PATENTSCHRIFT

(11) **EP 2 402 044 B1**
(45) Veröffentlichungstag und Bekanntmachung des Hinweises auf die Patenterteilung: **31.05.2017**
(21) Anmeldenummer: 11168170.6
(22) Anmeldetag: 31.05.2011
(51) Int. Cl.: A61L 31/02, A61L 31/08, A61L 31/14

(54) **Implantat und Verfahren zur Herstellung desselben**
Implant and method for producing the same
Implant et son procédé de fabrication

(30) Priorität: 29.06.2010 US 359365 P
(43) Veröffentlichungstag der Anmeldung: 04.01.2012
(73) Patentinhaber: Biotronik AG, 8180 Bülach (CH)
(72) Erfinder: Bayer, Ullrich, 18211 Admannshagen-Bargeshagen (DE); Rzany, Alexander, 90449 Nürnberg (DE)
(74) Vertreter: Randoll, Sören

(56) Entgegenhaltungen:
- EP-A2- 2 179 752
- ZHANG E ET AL: "Formation by ion plating of Ti-coating on pure Mg for biomedical applications", SCRIPTA MATERIALIA, ELSEVIER, AMSTERDAM, NL, vol. 53, no. 5, 1 September 2005 (2005-09-01), pages 523-527, XP025398701, ISSN: 1359-6462, DOI: 10.1016/J.SCRIPTAMAT.2005.05.009 [retrieved on 2005-09-01]
- WU G ET AL: "Improving corrosion resistance of titanium-coated magnesium alloy by modifying surface characteristics of magnesium alloy prior to titanium coating deposition", SCRIPTA MATERIALIA, ELSEVIER, AMSTERDAM, NL, vol. 61, no. 3, 1 August 2009 (2009-08-01) , pages 269-272, XP026116797, ISSN: 1359-6462, DOI: 10.1016/J.SCRIPTAMAT.2009.03.061 [retrieved on 2009-04-05]

## Beschreibung

Die Erfindung betrifft ein Verfahren zur Herstellung eines Implantats, insbesondere einer intraluminalen Endoprothese, mit einem Körper, wobei der Körper Magnesium oder eine Magnesiumlegierung aufweist sowie ein Implantat erhältlich oder erhalten durch ein derartiges Verfahren.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents, Befestigungselemente für Knochen, beispielsweise Schrauben, Platten oder Nägel, chirurgisches Nahtmaterial, Darmklammern, Gefäßclips, Prothesen im Bereich des Hart- und Weichgewebes sowie Ankerelemente für Elektroden, insbesondere von Schrittmachern oder Defibrillatoren zu verstehen.

Heutzutage werden als Implantate besonders häufig Stents eingesetzt, die zur Behandlung von Stenosen (Gefäßverengungen) dienen. Sie weisen einen Körper in der Form eines rohrförmigen oder hohlzylinderförmigen Grundgitters auf, das an beiden Längsenden offen ist. Das rohrförmige Grundgitter einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können.

Ein Ansatzpunkt zur Lösung dieses Problems besteht darin, den Stent bzw. andere Implantate aus einem biodegradierbaren Werkstoff zu fertigen.

Unter Biodegradation werden hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Körper biodegradierbarer Implantate geeignete Werkstoffe können beispielsweise Polymere oder Metalle enthalten. Der Körper kann dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Die vorliegende Erfindung bezieht sich auf Implantate mit einem metallischen biodegradierbaren Werkstoff basierend auf Magnesium oder Legierungen von Magnesium.

Es sind bereits Stents bekannt, die Beschichtungen mit verschiedenen Funktionen aufweisen. Derartige Beschichtungen dienen beispielsweise der Freisetzung von Medikamenten, der Anordnung eines Röntgenmarkers oder dem Schutz der darunter liegenden Strukturen.

Bei der Realisierung biodegradierbarer Implantate soll die Degradierbarkeit entsprechend der angestrebten Therapie bzw. der Anwendung des jeweiligen Implantats (koronar, intracranial, renal etc.) gesteuert werden. Für viele therapeutische Anwendungen ist beispielsweise ein wichtiger Zielkorridor, dass das Implantat in einem Zeitraum von vier Wochen bis sechs Monaten seine Integrität verliert. Hierbei wird unter Integrität, d. h. mechanischer Integrität, die Eigenschaft verstanden, dass das Implantat gegenüber dem undegradierten Implantat kaum mechanische Einbußen besitzt. Dies bedeutet, dass das Implantat mechanisch noch so stabil ist, dass zum Beispiel der Kollapsdruck lediglich geringfügig, d. h. höchstens auf 80% des Nominalwerts, abgefallen ist. Das Implantat kann somit bei vorhandener Integrität seiner Hauptfunktion, dem Aufhalten des Gefäßes, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers aufweist, oder im Fall eines Stents kaum angebrochene tragende Streben aufweist.

Als für den genannten Zielkorridor der Degradation besonders vielversprechend haben sich biodegradierbare Magnesium-Implantate, insbesondere Magnesium-Stents, erwiesen, die allerdings ihre mechanische Integrität bzw. Stützwirkung einerseits noch zu früh verlieren und andererseits in vitro und in vivo einen stark schwankenden Integritätsverlust aufweisen. Dies bedeutet, dass bei Magnesium-Stents der Kollapsdruck über die Zeit zu schnell verringert bzw. die Verringerung des Kollapsdrucks eine zu große Variabilität aufweist und damit zu unbestimmbar ist.

Im Stand der Technik sind bereits unterschiedliche Mechanismen der Degradationskontrolle von Magnesium-Implantaten beschrieben. Diese basieren beispielsweise auf anorganischen und organischen Schutzschichten oder deren Kombination, die dem humanen Korrosionsmilieu und den dort ablaufenden Korrosionsvorgängen einen Widerstand entgegensetzen. Bisher bekannte Lösungen zeichnen sich dadurch aus, dass Sperrschichtwirkungen erzielt werden, die auf einer räumlichen und möglichst defektfreien Trennung des Korrosionsmediums von dem metallischen Material, insbesondere dem metallischen Magnesium beruhen. So wird der Degradationsschutz durch verschieden zusammengesetzte Schutzschichten und durch definierte geometrische Abstände (Diffusionsbarrieren) zwischen dem Korrosionsmedium und dem Magnesiumgrundmaterial abgesichert. Andere Lösungen basieren auf Legierungsbestandteilen des biodegradierbaren Materials des Implantat-Körpers, welche den Korrosionsprozess durch Verschiebung der Lage in der elektrochemischen Spannungsreihe beeinflussen. Weitere Lösungen auf dem Gebiet der gesteuerten Degradation rufen durch das Aufbringen von physikalischen (z.B. lokale Querschnittsveränderungen) und/oder chemischen Veränderungen der Stent-Oberfläche (z.B. lokal chemisch unterschiedlich zusammengesetzte Multilayer) Sollbrucheffekte hervor. Mit den zuvor genannten Lösungen gelingt es jedoch meist nicht, die durch den Degradationsprozess eintretende Auflösung und die daraus resultierenden Stegbrüche in das geforderte Zeitfenster zu legen. Die Folge ist eine entweder zu früh oder zu spät einsetzende Degradation bzw. eine zu große Variabilität der Degradation des Implantats.

Ein weiteres Problem im Zusammenhang mit Beschichtungen ergibt sich auch daraus, dass Stents oder andere Implantate üblicherweise zwei Zustände annehmen, nämlich einen komprimierten Zustand mit einem kleinen Durchmesser und einen expandierten Zustand mit einem größeren Durchmesser. Im komprimierten Zustand kann das Implantat mittels eines Katheters in das zu stützende Gefäß eingeführt und an der zu behandelnden Stelle positioniert werden. Am Ort der Behandlung wird das Implantat dann beispielsweise mittels eines Ballonkatheters dilatiert. Aufgrund dieser Durchmesseränderung ist der Körper des Implantats hierbei einer mechanischen Belastung ausgesetzt. Weitere mechanische Belastungen des Implantats können während der Herstellung oder bei der Bewegung des Implantats im oder mit dem Gefäß, in das das Implantat eingesetzt ist, auftreten. Bei den genannten Beschichtungen ergibt sich somit der Nachteil, dass die Beschichtung während der Verformung des Implantats reißt (z.B. Bildung von Mikrorissen) oder auch teilweise entfernt wird. Hierdurch kann eine unspezifizierte lokale Degradation verursacht werden. Außerdem sind das Einsetzen und die Geschwindigkeit der Degradation von der Größe und der Verteilung der durch die Verformung entstehenden Mikrorisse abhängig, die als Fehlstellen schlecht kontrollierbar sind. Dies führt zu einer starken Streuung in den Degradationszeiten.

Aus den Druckschriften US 2008/0086195 A1 und WO 2008/045184 A1 ist eine medizinische Vorrichtung wie ein Katheter oder ein Stent bekannt, bei dem eine polymerfreie Beschichtung mittels eines plasmaelektrolytischen Prozesses (plasma electrolytic deposition PED) aufgebracht wird. Die plasmaelektrolytische Beschichtung wird dafür verwendet, zusätzlich Wirkstoffe in die Beschichtung einzubringen, welche ein Medikament oder ein therapeutisches Mittel beinhalten. Die plasmaelektrolytische Beschichtung umfasst eine plasmaelektrische Oxidation (plasma electrolytic oxidation PEO), eine Mikro-Lichtbogenoxidation (micro-arc oxidation MAO), eine Plasma-Lichtbogenoxidation (plasma-arc oxidation PAO), eine anodische Funkenoxidation (anodic spark oxidation) und eine elektrolytische Plasmasättigung (plasma electrolytic saturation PES). Die plasmaelektrolytische Beschichtung wird mittels Wechselspannung durchgeführt. Die plasmaelektrolytische Behandlung beinhaltet die Anwendung von verschiedenen elektrischen Potentialen zwischen der medizinischen Vorrichtung und einer Gegenelektrode, die eine elektrische Entladung (eine Funken- oder Lichtbogenplasmamikro-Entladung) an oder in der Nähe der Oberfläche der medizinischen Vorrichtung erzeugt, welche keine wesentliche Verlängerung der Degradationszeiten bewirkt. Das in den genannten Druckschriften angegebene Verfahren löst somit nicht das oben angegebene Problem.

Aus der Druckschrift DE 10 2008 042 602 sind ein Implantat sowie ein Verfahren zur Herstellung eines Implantats bekannt, bei dem ein Körper aus metallischem Material einer plasmachemischen Behandlung unterzogen wird. Durch die plasmachemische Behandlung wird auf der Körperoberfläche mit einer Frequenz von mindestens 1 kHz eine Schicht erzeugt, welche Phosphate, Hydroxide und Oxide des metallischen Materials, Strontiumcarbonat oder Strontiumphosphat aufweist. Durch die plasmachemische Behandlung wird zwar eine Schutzschicht auf der Oberfläche des Implantats erzeugt, welche zeitweise eine Degradation verhindert. Inzwischen hat sich jedoch gezeigt, dass eine wesentliche Erhöhung der Standzeit eines derartigen Implantats hierdurch nicht erreicht werden kann.

Folglich besteht die Aufgabe der vorliegenden Erfindung darin, ein Verfahren zur Herstellung eines Implantats anzugeben, das noch genauer eine Degradation des Implantats im gewünschten Zielkorridor ermöglicht. Dabei soll die Degradation zu einem besser kontrollierbaren Zeitpunkt stattfinden. Entsprechend besteht die Aufgabe der Erfindung außerdem darin, ein derartiges Implantat zu schaffen.

Die obige Aufgabenstellung wird gelöst durch ein Verfahren umfassend die folgenden Schritte:
a) Bereitstellen des Implantatkörpers,
b) Aufbringen einer metallischen Beschichtung mit dem Hauptbestandteil Titan auf mindestens einen Teil der Körperoberfläche,
c) plasmachemische Behandlung des mit der Beschichtung versehenen Teils der Körperoberfläche in einer wässrigen Lösung zur Herstellung einer plasmachemisch erzeugten Schicht mittels Anlegen einer das Plasma generierenden Mischspannung an den Körper des Implantats, wobei die Mischspannung eine ausreichende Energie dafür aufweist, dass sowohl die metallische Beschichtung als auch ein darunter liegender Bereich des Implantatkörpers zeitweise ionisiert wird und wobei die plasmachemische Behandlung der Körperoberfläche dadurch erfolgt, dass an den Körper eine gepulste Spannung angelegt wird, deren Peakspannung im überwiegenden Teil des Behandlungszeitraums mindestens etwa 90 V übersteigt und im Verlauf der Behandlung bis etwa 450 V ansteigt und die Energie im Bereich von 0,1 Ws bis 10 Ws liegt.

Bei dem erfindungsgemäßen Verfahren wird zunächst eine metallische Beschichtung mit Titan auf die Körperoberfläche des Implantatkörpers aufgebracht. Anschließend wird eine plasmachemische Behandlung derart durchgeführt, dass sowohl die metallische Beschichtung als auch ein darunter liegender Bereich des Materials des Implantatkörpers zeitweise ionisiert wird. Die plasmachemische Behandlung umfasst daher sowohl die metallische Beschichtung als auch eine darunter liegende Schicht der Körperoberfläche und bildet diese Schichten in eine neue, plasmachemisch erzeugte Schicht um.

Hierbei wird die Körperoberfläche in einem wässrigen Elektrolytsystem (wässrige Lösung) behandelt und die plasmachemischen Effekte entstehen direkt an der Oberfläche des Körpers des Implantats. Das für Mikrosekunden stabile Plasma an der Körperoberfläche erzeugt Reaktionsprodukte, welche zur Ausbildung der plasmachemisch erzeugten Schicht auf der Körperoberfläche führen. Als plasmachemisch erzeugte Schicht wird im Folgenden die Kombination der umgebildeten metallischen Beschichtung und der ebenfalls umgebildeten Schicht der darunter liegenden Körperoberfläche bezeichnet.

Der Körper des Implantats umfasst mindestens einen Teil des Implantats, vorzugsweise den Hauptteil des Implantats, welcher die mechanische Integrität des Implantats bewirkt. Die vorliegende Erfindung bezieht sich dabei insbesondere auf Implantate, deren Material des Körpers Magnesium als Hauptbestandteil enthält und vorzugsweise biodegradierbar ist.

Der Vorteil des erfindungsgemäßen Verfahrens besteht darin, dass durch die plasmachemische Behandlung auf der Körperoberfläche des Implantats in der plasmachemisch erzeugten Schicht Hydroxide, Oxide und Phosphate der beteiligten Metalle, insbesondere des Magnesiums und der in der zuvor aufgebrachten metallischen Beschichtung enthaltenen Metalle, gebildet werden. Ferner werden in dieser Schicht auch Legierungen der genannten Metalle erzeugt. Die plasmachemisch erzeugte Schicht stellt bei Kontakt mit der Körperflüssigkeit einen temporären Korrosionsschutz dar, der eine verzögerte Degradation bewirkt.

Ein ganz wesentlicher Vorteil des erfindungsgemäßen Verfahrens gegenüber dem Stand der Technik besteht darin, dass durch die hohe Energie der das Plasma generierenden Mischspannung (auch Impulsspannung genannt) sowohl die metallische Beschichtung als auch ein darunter liegender Bereich des Implantatkörpers zeitweise ionisiert werden. Hierbei steigt die Spannung von anfänglich 0 V über einen Zeitraum von ca. 1 µs auf eine Peakspannung an und fällt dann parabolisch innerhalb der darauf folgenden 4 µs ab. Die Peakspannung wird von Puls zu Puls bis auf einen Maximalwert erhöht, der bei etwa 450 V liegt. Durch die Trägheit des Systems (Elektrolyt, Elektroden, mechanische Trägheit der Ladungsträger im Elektrolyt) geht die Spannung nach Durchlaufen der Peakspannung jedoch nicht mehr auf Null zurück sondern lediglich auf einen Minimalwert. Danach steigt die Spannung wieder bis zur nächsten Peakspannung an usw.. Durch das so generierte Plasma wird nicht nur die metallische Beschichtung ionisiert sondern auch das Implantatkörper-Material, das direkt unterhalb der metallischen Beschichtung angeordnet ist, bis in eine bestimmte, von der konkreten Energie der Mischspannung abhängigen Tiefe. Hierdurch entsteht ein enger Stoffschluss zwischen dem Implantatkörper und der plasmachemisch erzeugten Schicht, welcher zu einer hohen Adhäsionsfestigkeit der auf der Implantatkörper-Oberfläche angeordneten Schicht führt. Die plasmachemisch erzeugte Schicht mit den Bestandteilen der metallischen Beschichtung wird gewissermaßen in die Oberfläche des Implantatkörpers einlegiert. Hieraus folgt, dass die plasmachemisch erzeugte Schicht auch bei hoher mechanischer Beanspruchung des erfindungsgemäßen Implantats, beispielsweise bei der Stentdilatation, nicht von dem Implantatkörper delaminiert. Die Degradation erfolgt daher kontrollierbarer, da keine rein metallischen Flächen des Implantatkörpermaterials freigelegt werden.

Die Energie ist eine Produkt aus der Badspannung U, dem Strom I und der Zeit bzw. ein Produkt aus Spannung, Stromdichte, Fläche und Pulslänge. Vorzugsweise werden zur plasmachemischen Behandlung Energien im Bereich von 0,1 Ws bis 10 Ws verwendet, besonders bevorzugt für Magnesiumstents ist der Bereich zwischen 0,5 Ws und 5 Ws Energie. Die angegebenen Energiewerte gelten insbesondere für Implantate mit einer Gesamtoberfläche in der Größenordnung von ca. 100 mm².

Beispielsweise ergibt sich bei einer mittleren Badspannung von 300 V (Maximalwert der Peakspannung beträgt hierbei 400 V), einer Stromdichte von 1 A/dm² = 10 mA/cm², einer Pulsdauer von 5 µs und 1000 µs Pulspause und einer Beschichtungszeit von 1 Minute folgende energetische Beziehung:
Implantatoberfläche eines Stents ca. 100 mm² = 0,01 dm²
Beschichtungsenergie W = U · I · t_{eff}.
= 300 V · 0,01 dm²· 0,005 A/dm² · 60s
= 0,9 Ws
Das bedeutet, dass die Oberfläche eines Implantates wie z.B. eines Stents mit einer Oberfläche von ca. 100 mm² mit einer Beschichtungsenergie von ca. 0,9 Wattsekunden plasmachemisch oberflächenbehandelt wird.

Die mittels des erfindungsgemäßen Verfahrens hergestellte degradationshemmende plasmachemische erzeugte Schicht weist außerdem verfahrensbedingt Poren auf. Die vor und nach den Degradationstests durchgeführten oberflächenanalytischen Untersuchungen haben gezeigt, dass die mit der plasmachemischen Behandlung einhergehende Hydroxidbildung zu einer lokal begrenzten Versiegelung der Porengründe führt.

Die Versiegelung der Porengründe kann beispielsweise anhand des mehrstufigen Verlaufs des pH-Werts des Elektrolyten in einem Korrosionstest (zum Beispiel in künstlichem Plasma) nachgewiesen werden. In den ersten sieben Tagen steigt der pH-Wert erwartungsgemäß von 7,4 auf 8,5 bis 8,7 an. Nach einem nach sieben Tagen durchgeführten Medienwechsel erhöht sich der pH-Wert in überraschender Weise von 7,4 nur noch auf 8,0. Dieses Verhalten zeigt eine geringere chemische Aktivität des metallischen Grundmaterials (insbesondere des Magnesiums) mit dem korrosiven Medium. Dieser Effekt geht einher mit einem Stopp der Querschnittsverjüngung des Materials des Implantat-Körpers, der bereits nach sieben Tagen im metallographischen Querschliff zu verzeichnen ist. Dieser Effekt kann beispielsweise dadurch erklärt werden, dass Hydroxide des metallischen Materials die Poren in der ersten Schicht ausfüllen und damit der Kontakt des Elektrolyten zum metallischen Grundmaterial unterbunden wird. Durch diesen Selbstheilungseffekt wird die Standzeit der Implantate unter Körpermilieubedingungen wesentlich erhöht.

Außerdem weist die verfahrensbedingt poröse Struktur der plasmachemisch erzeugten Schicht ein hohes plastisches Verformungsvermögen auf. Beispielsweise werden die beim Dilatieren eines Stents entstehenden Mikrorisse durch Energieakkumulation beziehungsweise -dissipation in den zu den Mikrorissen benachbarten Poren gestoppt. Es kommt somit nicht zu einer Delamination der plasmachemisch erzeugten Schicht.

Falls gewünscht, ist noch eine weitere, z.B. polymere Beschichtung auf die plasmachemisch erzeugte Schicht aufzubringen, so ergibt sich aufgrund der Porenstruktur eine gute Haftung der Beschichtung auf der plasmachemisch erzeugten Schicht.

Außerdem kann die Porenstruktur der plasmachemisch erzeugten Schicht als Stoffreservoir fungieren, etwa für pharmazeutisch aktive Substanzen, welche als Nano- oder Mikropartikel eingelagert werden können oder für Substanzen, die als Gleitmittel zur Verringerung des Reibungskoeffizienten im Katheter verwendbar sind. Ferner können knochenwachstumsfördernde Substanzen wie Kalziumphosphate in die Porenstruktur eingelagert werden sowie temporär wirkende Kontrastmittel oder zellwachstumshemmende radioaktive Substanzen.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Umgebung des Implantats hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können beispielsweise aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, der Taxole oder Taxane, hier vorzugsweise Paclitaxel, oder Sirolimus, bestehen.

Aufgrund des anodischen Wirkprinzips der plasmachemischen Beschichtung besteht zudem keine Gefahr der Wasserstoffversprödung. Auch die Gefahr einer mechanischen Beschädigung besteht nicht, da das Aufrauen der Oberfläche nicht mechanisch erfolgt.

Ein weiterer Vorteil des erfindungsgemäßen Verfahrens besteht darin, dass nicht entfernbare Oberflächenkontaminationen des Grundmaterials durch die plasmachemisch erzeugte Schicht absorbiert werden und somit den Degradationsprozess nicht zusätzlich beeinflussen. Zudem werden aus der Oberfläche herausragende, teilweise scharfkantige Ausscheidungen des Implantat-Körpers (wie zum Beispiel nicht gelöste Legierungsbestandteile) abgedeckt. Daraus folgt auch eine erhöhte Hämo- bzw. Biokompatibilität.

Aufgrund der Existenz der mittels des erfindungsgemäßen Verfahrens hergestellten plasmachemisch erzeugten Schicht vereinfachen sich zudem die Lager- und Transportbedingungen für nach dem erfindungsgemäßen Verfahren hergestellten Implantate, da die Stabilität eines solchen Implantats gegenüber Degradation höher ist als bei unbeschichteten Implantaten.

In einem bevorzugten Ausführungsbeispiel beträgt eine erste Schichtdicke der metallischen Beschichtung nach Abschluss von Schritt b) etwa 0,1 µm bis etwa 3 µm. Die genannte Dicke der metallischen Beschichtung ist derart gewählt, dass die gesamte metallische Beschichtung und auch das darunter liegende Material des Implantatkörpers mittels realisierbarer plasmachemischer Oxidationsanlagen kurzzeitig ionisiert werden kann. Außerdem kann die Degradationsgeschwindigkeit durch Variation der Schichtdicke gesteuert werden. Hierdurch eröffnet sich auch die Möglichkeit, die Degradationsdauer des Implantats an den spezifischen Implantationsort anzupassen (koronar, intracranial, renal etc.)

Es ist weiterhin von Vorteil, wenn die metallische Beschichtung mittels einer ionischen Flüssigkeit aufgebracht wird.

Alternativ können auch PVD- und CVD-Verfahren (wie z.B. Sputtern oder thermisches Aufdampfen) zum Aufbringen der metallischen Beschichtung verwendet werden. Allerdings dürfen diese beim Einsatz von Magnesium als Implantatmaterial eine Temperatur von ca. 450°C bei einer Behandlungszeit von 60 min nicht überschreiten. Andernfalls würden sich irreversible Änderungen der mechanischen Werkstoffeigenschaften ergeben (Entfestigung). Die Beschichtung mittels PVD- oder CVD-Verfahren haben jedoch gegenüber einer Beschichtung mittels ionischer Flüssigkeit den Nachteil, dass keine einheitliche Bedeckung der häufig kompliziert geformten Implantatoberflächen stattfindet. Deshalb sind diese Verfahren insbesondere für Implantate geeignet, die einfach geformte Oberflächen aufweisen.

Demgegenüber kann die Beschichtung mittels ionischer Flüssigkeiten für komplizierter geformte Implantate angewendet werden. Ein solches Beschichtungsverfahren erlaubt die Realisierung einer einheitlichen Schichtdicke auch bei komplizierten Formen (z.B. im Inneren von Stents) und trägt damit maßgeblich zu einem uniformen Degradationsverhalten nach der plasmachemischen Behandlung bei.

In einem weiteren Ausführungsbeispiel wird das Implantat nach der plasmachemischen Beschichtung in einem Lösungsmittel, vorzugsweise destilliertem H₂O, gespült und danach vorzugsweise bei einer Temperatur von mindestens etwa 80°C, besonders bevorzugt mindestens etwa 100°C, getrocknet, wobei die Trocknung vorzugsweise in einem Umluftofen durchgeführt wird. Diese Vorgehensweise bewirkt das rückstandslose Entfernen von Verbindungen des plasmachemischen Elektrolyten von der Implantatoberfläche. Die Trocknung bei erhöhten Temperaturen stellt auch sicher, dass eventuell durch Kapillarwirkung länger anhaftende Elektrolytreste (z.B. in der filigranen Oberflächenform eines Stents) keine unerwünschten Reaktionen mit der Implantatoberfläche eingehen.

In einem bevorzugten Ausführungsbeispiel enthält die wässrige Lösung Sr²⁺ Ionen, die vorzugsweise in einer Konzentration von jeweils 0,05 Mol/l bis 2,0 Mol/l Sr²⁺ in der wässrigen Lösung enthalten sind. Hierdurch werden Strontiumverbindungen in der plasmachemisch erzeugten Schicht gebildet. Diese sind vorteilhaft, da insbesondere Strontiumcarbonat kaum wasserlöslich ist und somit einen die Degradation besonders hemmenden Schichtbestandteil in der Oberflächenschicht des Implantats bildet. Zudem kann das in der Beschichtung enthaltene Strontiumcarbonat insbesondere bei cranialen Anwendungen eine arzneimittelähnliche Wirkung gegen Zerebralsklerose entfalten.

In einem bevorzugten Ausführungsbeispiel des erfindungsgemäßen Verfahrens sind in der wässrigen Lösung, die für die plasmachemische Behandlung verwendet wird, zusätzlich ein oder mehrere Ionen ausgewählt aus der Gruppe der Phosphate, Carbonate, Hydroxide und Silikate enthalten.

Enthält die wässrige Lösung zur plasmachemischen Behandlung Phosphationen, so bilden sich neben den Oxiden und Hydroxiden in der plasmachemisch erzeugten Schicht auch Phosphate des Körpermaterials aus, die für eine bessere biologische Verträglichkeit des Implantatmaterials, insbesondere der Beschichtung, sorgen. Dabei entstammen die Phosphationen der Zugabe von Kaliumdihydrogenphosphat und/oder Dikaliumhydrogenphosphat und/oder Kaliumphosphat und/oder Natriumdihydrogenphosphat (Dihydrat) und/oder Heptahydrat und/oder Dodecahydrat in den wässrigen Elektrolyten. Der bevorzugte Konzentrationsbereich liegt dabei zwischen 5 g/l und 200 g/l der zugegebenen Verbindung in der wässrigen Lösung. Eine besonders bevorzugte Konzentration beträgt zwischen 50 und 100 g/l Kaliumdihydrogenphosphat.

Um den pH-Wert des Elektrolyten (wässrigen Lösung) konstant zu halten, ist bevorzugt in der wässrigen Lösung ein Puffer, vorzugsweise Kaliumdihydrogenphosphat und/oder Natriumdihydrogenphosphat enthalten.

In einem weiteren bevorzugten Ausführungsbeispiel wird der Implantat-Körper vor dem Auftragen der metallischen Beschichtung oder der plasmachemischen Behandlung elektrochemisch behandelt, vorzugsweise elektrochemisch poliert. Hierdurch werden Verunreinigungen auf der Oberfläche des Implantat-Körpers entfernt, so dass das Aufbringen der metallischen Beschichtung oder die plasmachemische Behandlung an einer definierten Oberfläche erfolgt. Wichtig ist die Freiheit von Oberflächenkontaminationen, die sonst zu einer schlechten Haftung der plasmachemisch erzeugten Schicht führen würde. Das Elektropolieren führt zu kontaminationsarmen Deckschichten Aufgrund großer Materialabtragseffekte (Tiefenwirkung).

Bevorzugt erfolgt die plasmachemische Behandlung des Implantat-Körpers dadurch, dass an den Körper eine gepulste, vorzugsweise positive, Spannung angelegt wird, deren Amplitude (Peakspannung) im überwiegenden Zeitraum der Behandlung mindestens etwa 90 Volt übersteigt, besonders bevorzugt mindestens etwa 100 Volt übersteigt und welche vorzugsweise im Verlauf der Behandlung ansteigt. Der Spannungsverlauf entspricht des oben mit Misch- oder Impulsspannung bezeichneten Verlaufs.

Durch diese hohen gepulsten Spannungen mit einer Pulslänge von vorzugsweise maximal etwa 50 Mikrosekunden, besonders bevorzugt etwa 5 Mikrosekunden, werden an der Oberfläche des Implantat-Körpers über Mikrosekunden Plasmen erzeugt, welche zur Reaktion des metallischen Materials des Implantatkörpers mit dem Elektrolyten führen. Zwischen den Spannungspulsen folgt eine Ruhephase von vorzugsweise ca. 1000 Mikrosekunden. Die aufzubringende Energie in Form der Spannungspulse beträgt bei einer Implantatoberfläche von 100 mm² mindestens ca. 0,5 Ws. Bei Implantatoberflächen mit einer abweichenden Größe verändert sich die Mindestenergie proportional zum Flächeninhalt der Oberfläche.

Bevorzugt wird der plasmachemische Prozess mit einer Stromdichte von mindestens etwa 3 mA/cm², vorzugsweise mindestens etwa 5 mA/cm² durchgeführt.

In einem weiteren, besonders bevorzugten Ausführungsbeispiel wird ein Implantat mit einer metallischen Beschichtung, welche Titan enthält und vorzugsweise kein Aluminium enthält, nach der plasmachemischen Behandlung einer Nachbehandlung des Körpers des Implantats in einer wässrigen, stark basischen Natriumhydroxidlösung zur Erzeugung einer Porengrundschicht unterzogen, wobei die Nachbehandlung unter Beaufschlagung von Ultraschall und/oder Einblasen von Argon und/oder Stickstoff durchgeführt wird. Der pH-Bereich liegt dabei vorzugsweise - je nach NaOH-Konzentration - zwischen etwa 11 und etwa 13,8. Die Temperatur der Nachbehandlungslösung liegt bevorzugt zwischen Raumtemperatur und 70°C. Die bevorzugte Verweildauer des Implantatkörpers in der Natriumhydroxidlösung beträgt zwischen 5 s und 180 s.

Durch die Nachbehandlung erfolgt eine Versiegelung der aus der Sicht der Korrosionsbeständigkeit bzw. Durchlässigkeit kritischen Porengründe der durch die plasmachemische Beschichtung erzeugten Schicht, so dass die Porengrundschicht im Wesentlichen in den Porengründen vorliegt. Durch die Nachbehandlung werden lediglich die Porengründe und nicht die kompletten Poren verschlossen, so dass der positive Effekt der durch die plasmachemische Behandlung aufgerauten Oberfläche und der damit verbundenen hohen Adhäsion zu polymeren Deckschichten erhalten bleibt. Hierdurch wird eine erhöhte Korrosionsbeständigkeit des Implantats erzielt, die zu einer Degradation und Integritätsverlust in einem für viele Behandlungen interessanten Zeitfenster von 3 bis 12 Monaten führt.

Der bei diesem bevorzugten Ausführungsbeispiel erfindungsgemäß erzielte, auf der Bildung von Hydroxiden (z.B. von Titanhydroxiden) beruhende Versiegelungseffekt des Porengrundes bewirkt zudem einen sehr vorteilhaften Rissstopmechanismus. Dieser entsteht Aufgrund des Unterschieds der mechanischen Konsistenz des Materials der porösen Deckschicht einerseits und dem Material des versiegelten Porengrundes andererseits. Er beruht auf der hohen Aufnahmefähigkeit der Pore für die Rissenergie.

Mikrorisse entstehen beispielsweise im Moment der Dilatation von Stents oder anderen Implantaten, wenn diese in mikroskopischen Bereichen über ihr Plastifizierungsvermögen hinaus beansprucht werden und führen zu einer unkontrollierten und deshalb unerwünschten Degradation des Grundmaterials des Implantatkörpers. Die bei dem bevorzugten Ausführungsbeispiel in der plasmachemisch erzeugten, aus größtenteils röntgenamorphen Oxiden, Hydroxiden und/oder Phosphaten des Grundmaterials bestehenden plasmachemisch erzeugten Schicht auftretenden Risse, werden in den Porengründen aufgefangen. Dies beruht einerseits auf der ruhenden und damit Rissenergie akkumulierenden Geometrie der Pore an sich und andererseits auf der in dem Porengrund angeordneten und im Vergleich zur übrigen Schichtmatrix zähen Hydroxid (z.B. Titanhydroxid). Die Hydroxidschicht auf dem Porengrund wird durch die erfindungsgemäße Nachbehandlung vorteilhaft verstärkt, so dass der beschriebene Rissstoppmechanismus besonders wirksam funktioniert. Hierdurch wird eine hohe Schadenstoleranz bei mechanischer Beanspruchung erzielt, so dass die Degradation gleichmäßiger stattfindet. Die im Wesentlichen auf dem Grund der Poren der plasmachemisch erzeugten Schicht entstehende dritte Schicht weist in der Regel eine Dicke von einigen 10 nm auf.

Ein weiterer Vorteil der erfindungsgemäßen Kombination der plasmachemisch erzeugten Schicht und der Porengrundschicht ist, dass keine Blasenbildung und damit bei fortgeschrittener Degradation aufgrund von Semipermeabilität des Schichtverbunds kein Lumenverlust auftritt bzw. die Blasenbildung reduziert ist.

In einem weiteren bevorzugten Ausführungsbeispiel wird nach der plasmachemischen Behandlung oder nach der oben beschriebenen Nachbehandlung auf die plasmachemisch erzeugte Schicht und/oder die Porengrundschicht eine weitere, vorzugsweise ein Polymer enthaltende Schicht (im Folgenden vierte Schicht genannt), besonders bevorzugt enthaltend mindestens ein Polymer der Gruppe Parylene und PLA (Polylactid), insbesondere PLLA (Poly-L-Lactid), aufgebracht. Bevorzugte Schichtdicken der vierten Schicht liegen zwischen etwa 0,5 und etwa 10 µm. Durch eine derartige Schichtkombination kann die Degradationszeit des Implantats nochmals wesentlich erhöht werden. Vorteilhaft wirkt sich auch die hohe Spaltgängigkeit von Parylene aus, so dass auch eine tiefgehende Penetration der Poren der plasmachemisch erzeugten Schicht und/oder die Porengrundschicht bis hin zu den Porengründen durch Parylene erfolgt. Die für PLA und Parylene, insbesondere Parylene N, charakteristischen Permeationseigenschaften für Wasser, chloridhaltige Lösungen und Wasserstoff sorgen in Verbindung mit der darunter liegenden plasmachemisch erzeugten und ggf. an den Porengründen versiegelten Oberfläche für ein steuerbares Degradationsverhalten des Implantats. Dieses zeichnet sich auch durch einen über den Implantatquerschnitt uniformen langsamen Korrosionsfortschritt aus. Zudem leistet die vierte Schicht einen zusätzlichen Beitrag zur Vermeidung bzw. Behinderung des Rissfortschritts bei mechanischer Belastung und verhindert partielle Schichtablösungen. Insbesondere bevorzugt ist, wenn die vierte Schicht aus Parylene N oder PLLA L210 besteht.

Hierbei ist Parylene die Bezeichnung für vollständig lineare, teilkristalline, unvernetzte aromatische Polymere. Die verschiedenen Polymere besitzen unterschiedliche Eigenschaften und lassen sich in vier Grundtypen einteilen, nämlich Parylene C, Parylene D, Parylene N und Parylene F. Für die Anwendung als vierte Schicht im erfindungsgemäßen Schichtverbund wird vorzugsweise Parylene N verwendet.

Die unter der vierten Schicht angeordnete plasmachemisch erzeugte Schicht und/oder Porengrundschicht führt/führen Aufgrund ihrer porösen Struktur zu einem hohen Haftungsvermögen der vierten Schicht, so dass eine sonst vorzuschaltende Primerbehandlung überflüssig wird.

Die obige Aufgabenstellung wird ferner gelöst durch ein Implantat erhältlich durch ein oben beschriebenes erfindungsgemäßes Verfahren. Ein derartiges Implantat weist die oben im Zusammenhang mit dem erfindungsgemäßen Herstellungsverfahren angegebenen Vorteile auf. Erfindungsgemäß weist die metallische Beschichtung nach dem Schritt b) eine erste Schichtdicke auf und eine zweite Schichtdicke der plasmachemisch erzeugten Schicht nach Schritt c) ist größer als die erste Schichtdicke. Dies ist dadurch begründet, dass die plasmachemisch erzeugte Schicht aus einer Umbildung der metallischen Beschichtung und einer Umbildung der darunterliegenden oberflächennahen Schicht des Implantatkörpers durch das Plasma resultiert.

Vorzugsweise hat die mittels der plasmachemischen Behandlung erzeugte Schicht eine Dicke (zweite Schichtdicke) von mindestens 0,3 µm, vorzugsweise mindestens 1 µm.

Oben wurde bereits beschrieben, dass die plasmachemisch erzeugte Schicht Poren aufweist, wobei vorzugsweise, insbesondere bei einer vorstehend beschriebenen Behandlung des Implantats in einer Natriumhydroxidlösung, auf dem Porengrund als Porengrundschicht ein Hydroxid des metallischen Materials oder der metallischen Materialien des Implantat-Körpers und/oder der metallischen Beschichtung gebildet ist.

Bevorzugt enthält die plasmachemisch erzeugte Schicht mindestens eine Verbindung ausgewählt aus der Gruppe enthaltend
- Legierungen aus dem Material des Implantatkörpers und des Materials der metallischen Schicht, insbesondere Legierungen aus Magnesium und Titan, sowie
- Phosphate, Hydroxide und Oxide des Materials des Implantatkörpers und des Materials der metallischen Schicht, insbesondere Phosphate, Hydroxide und Oxide des Magnesiums und des Titans.

Hierbei wird ein Zusammensetzungsgradient von der Oberfläche des Implantats in die Tiefe bis zum Übergang der plasmachemischen erzeugten Schicht in das Material des Implantatkörpers beobachtet.

Der Vorteil von Titan als Bestandteil der metallischen Beschichtung und somit auch als Bestandteil der plasmachemischen erzeugten Schicht besteht darin, dass diese Materialien sich mit wesentlich geringerem Energieaufwand bei der plasmachemischen Behandlung oxidieren lassen als andere metallische Materialien. Dies bedeutet, dass die Tiefenwirkung der plasmachemischen Behandlung, das heißt die Fähigkeit des Plasmas, das Material des Implantatkörpers zu erreichen und dieses zu oxidieren und mit dem Metall der metallischen Beschichtung eine stoffschlüssige Verbindung herzustellen, mit geringeren Energieeinträgen verbunden ist. Dies lässt sich vor allem durch den wesentlich geringeren Schmelzpunkt von Titan (ca. 1670°C) im Vergleich zu Metallen wie Tantal (ca. 3000°C) oder Niob (ca. 2470°C) erklären.

Ein weiterer Vorteil des erfindungsgemäßen Implantats besteht darin, dass durch die Oberflächenfunktionalisierung der Körper des Implantats in der Anfangsphase der Verweilzeit des Implantats im Körper dieser nicht sofort mit einer Metalloberfläche in Kontakt kommt. Damit sinkt die Gefahr von Inflammationen und die Endothelzellen haben zumindest in den ersten Stunden der Verweildauer des Implantats im Körper keinen oder nur geringen Kontakt mit Metallionen.

Ferner wird das Umformvermögen des Implantats, z. B. bei der Dilatation, verbessert. Dies liegt daran, dass ein Gradient des E-Moduls von der äußeren Oberfläche des erfindungsgemäßen Implantats, also von der äußeren Oberfläche der plasmachemisch erzeugten Schicht, nach innen vorliegt. Dadurch werden die mechanischen Spannungen zwischen der Implantatoberfläche und der neutralen Phase im Inneren des Bauteils wesentlich verringert.

Die erfindungsgemäßen Verfahren werden nachfolgend anhand von Beispielen und Figuren erläutert. Dabei bilden alle abgebildeten und/oder beschriebenen Merkmale den Gegenstand der Erfindung, unabhängig von ihrer Zusammenfassung in den Ansprüchen oder deren Rückbeziehung.

Es zeigen schematisch:

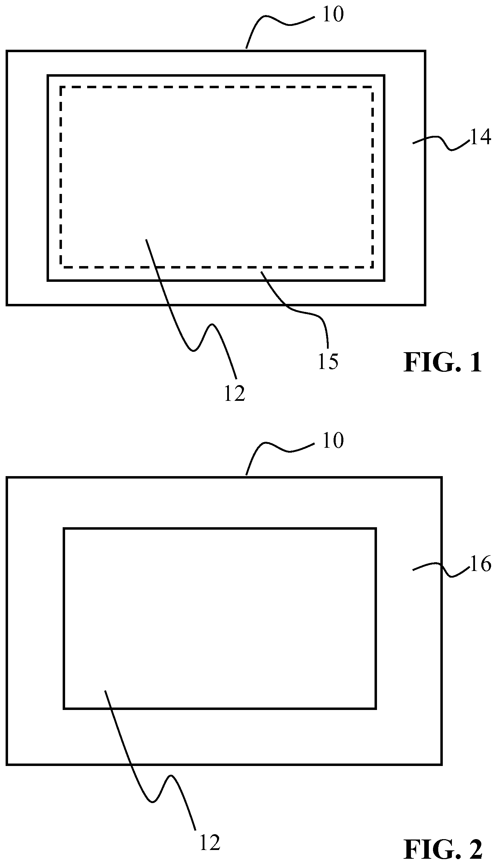

- Fig. 1: einen Querschnitt einer Strebe eines erfindungsgemäßen Implantats (Stent) nach Schritt b) des erfindungsgemäßen Verfahrens,
- Fig. 2: einen Querschnitt einer Strebe des erfindungsgemäßen Implantats gemäß Figur 1 nach Schritt c) des erfindungsgemäßen Verfahrens und

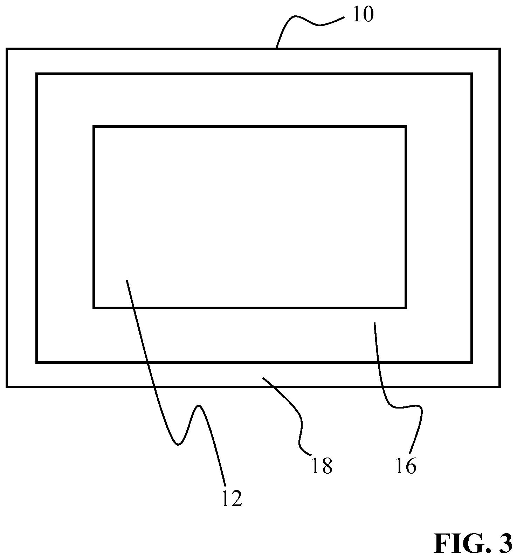

- Fig. 3: einen Querschnitt einer Strebe des erfindungsgemäßen Implantats gemäß Figur 2 mit einer zusätzlichen Schicht enthaltend ein Polymer.

### Beispiel 2:

Der Stent-Körper 12 aus einer Magnesiumlegierung WE43 wird mittels ionischer Flüssigkeit mit einer 1 µm dicken Schicht aus Titan beschichtet. Anschließend wird eine plasmachemische Oxidation in einem wässrigen, phosphathaltigen Elektrolyten durchgeführt. Hierdurch entsteht eine etwa 3 µm dicke plasmachemisch erzeugte Schicht mit den oben beschriebenen Eigenschaften. Diese enthält beispielsweise auch Titandioxid (TiO₂), das dem Stent eine höhere Biokompatibilität verleiht als ein am Stent aus der gleichen Legierung, der lediglich elektropoliert ist.

Der Elektrolyt ist eine ionische Flüssigkeit und besteht aus 1-Methyl-3-Butyl-Imidazolium-bis(trifluoromethylsulfon) ([BMIm]BTA). Titan(IV)chlorid ist in dieser Flüssigkeit sehr gut löslich. Für die Abscheidung wird eine Konzentration von 0,24 mol·1⁻¹ TiCl₄ eingestellt. Die Beschichtung erfolgt bei Raumtemperatur. Die Beschichtungszeit beträgt etwa 4 bis 6 Stunden. Die potentiostatische Polarisierung bei Verwendung von Gegen- und Referenzelektrode aus Platin beträgt -1.9 V gegen Ferrocen.

Ein gemäß Beispiel 2 beschichteter Stent (ohne Polymer-Beschichtung) weist eine um den Faktor 1,5 höhere Standzeit gegenüber der Referenz auf. Dies bedeutet, dass ein Stent der in einem mit HEPES (2-(4-(2-Hydroxyethyl)-1-piperazinyl)-ethansulfonsäure) gepuffertem künstlichem Plasma (pH = 7,4) über 42 Tage gelagert wurde, noch ca. 70% des ursprünglichen Kollapsdrucks aufweist. Hierbei ist die Zusammensetzung des Plasmas wie folgt (wässrige Lösung):

| Im Plasma enthaltene Verbindung | Konzentration[g/l] |
|---|---|
| NaCl | 6,8 |
| CaCl₂ | 0,2 |
| KCl | 0,4 |
| MgSO₄ · 7H₂O | 0,205 |
| NaHCO₃ | 2,2 |
| Na₂HPO₄ | 0,126 |
| NaH₂PO₄ · H₂O | 0,03 |

Nicht beschichtete Referenzstents bestehend aus der Magnesiumlegierung WE43 ohne weitere Beschichtung zeigen zu diesem Zeitpunkt nur noch Kollapsdruckwerte von maximal 40% des Ausgangswertes nicht ausgelagerter Stents. Metallographische Untersuchungen belegen außerdem, dass der verbleibende metallische Restquerschnitt (gemessen im Querschliff) einer Strebe eines erfindungsgemäß hergestellten Stents nach diesem Zeitraum noch bei ca. 60% des Ausgangsquerschnitts vor der Auslagerung beträgt. Nicht beschichtete Referenzstents weisen dagegen nach diesem Zeitraum nur noch 30% bis 40% metallischen Restquerschnitt auf.

In vivo Untersuchungen mit nach obigem Beispiel 2 hergestellten Stents zeigten, dass keine inflammatorischen Effekte im Kontakt mit körpereigenen Zellen auftreten. Dies trifft auf alle Beschichtungsvarianten mit Titan und jeweils auch mit einer zusätzlichen polymerer Deckschicht zu. Es wurden keine Unterschiede zwischen der erfindungsgemäßen Lösung und unbeschichteten Implantaten aus der Magnesium-Legierung WE 43 gefunden.

Histologische Untersuchungen zeigten, dass die gemäß Beispiel 2 beschichteten Implantate, welche entweder mit einer Polymerbeschichtung versehen sind oder keine Polymerbeschichtung haben, neben der verzögerten Degradation auch eine über die gesamte Abmessung des Implantates gleichmäßigere Abnahme des metallischen Restanteils aufweisen als bei unbeschichteten Implantaten aus der Magnesium-Legierung WE43 und somit die Stützwirkung länger erhalten bleibt. Dies vereinfacht die Kalkulation der Abnahme der mechanischen Belastungsfähigkeit mit fortschreitender Implantationszeit und macht diese verlässlicher.

### Bezugszeichenliste

- 10: Strebe (Strut) des Stents
- 12: Körper des Stents
- 14: metallische Beschichtung
- 15: unterhalb der metallischen Beschichtung 14 liegender Volumenbereich des Stentkörpers 12, der bei der plasmachemischen Oxidation zusammen mit der metallischen Beschichtung 14 ionisiert wird
- 16: plasmachemisch erzeugte Schicht
- 18: Polymer-Schicht

## Patentansprüche

1. Verfahren zur Herstellung eines Implantats, insbesondere einer intraluminalen Endoprothese, mit einem Körper (12), wobei der Körper Magnesium oder eine Magnesiumlegierung aufweist, umfassend die folgenden Schritte:
a) Bereitstellen des Implantatkörpers (12),
b) Aufbringen einer metallischen Beschichtung (14) mit dem Hauptbestandteil Titan auf mindestens einen Teil der Körperoberfläche,
c) plasmachemische Behandlung des mit der Beschichtung versehenen Teils der Körperoberfläche in einer wässrigen Lösung zur Herstellung einer plasmachemisch erzeugten Schicht (16) mittels Anlegen einer das Plasma generierenden Mischspannung an den Körper des Implantats, wobei die Mischspannung eine ausreichende Energie dafür aufweist, dass sowohl die metallische Beschichtung (14) als auch ein darunter liegender Bereich (15) des Implantatkörpers (12) zeitweise ionisiert wird und wobei die plasmachemische Behandlung der Körperoberfläche dadurch erfolgt, dass an den Körper eine gepulste Spannung angelegt wird, deren Peakspannung im überwiegenden Teil des Behandlungszeitraums mindestens 90 V übersteigt und im Verlauf der Behandlung bis 450 V ansteigt und die Energie im Bereich von 0,1 Ws bis 10 Ws liegt.

2. Verfahren nach Anspruch 1, **dadurch gekennzeichnet, dass** eine erste Schichtdicke der metallischen Beschichtung (14) nach Abschluss von Schritt b) etwa 0,1 µm bis etwa 3 µm beträgt.

3. Verfahren nach einem der vorhergehenden Ansprüche **dadurch gekennzeichnet, dass** die metallische Beschichtung (14) mittels einer ionischen Flüssigkeit, mittels eines PVD-Verfahrens oder mittels eines CVD-Verfahrens aufgebracht wird.

4. Verfahren nach einem der vorhergehenden Ansprüche, **dadurch gekennzeichnet, dass** das Implantat nach Schritt c) in einem Lösungsmittel, vorzugsweise mittels destilliertem H₂O, gespült und anschließend vorzugsweise bei einer Temperatur von mindestens 80°C, besonders bevorzugt mindestens 100°C, getrocknet wird, wobei die Trocknung vorzugsweise in einem Umluftofen durchgeführt wird.

5. Verfahren nach einem der vorhergehenden Ansprüche, **dadurch gekennzeichnet, dass** die wässrige Lösung Sr²⁺Ionen enthält, die vorzugsweise in einer Konzentration von jeweils 0,05 Mol/l bis 2,0 Mol/l Sr²⁺ in der wässrigen Lösung enthalten sind.

6. Verfahren nach einem der vorhergehenden Ansprüche, **dadurch gekennzeichnet, dass** die wässrige Lösung zusätzlich ein oder mehrere Ionen ausgewählt aus der Gruppe der Phosphate, Carbonate und Silikate aufweist und/oder dass in der wässrigen Lösung ein Puffer, vorzugsweise Kaliumdihydrogenphosphat und/oder Natriumdihydrogenphosphat enthalten ist.

7. Verfahren nach einem der vorhergehenden Ansprüche, **dadurch gekennzeichnet, dass** die Spannung in Schritt c) nach Durchlaufen der Peakspannung auf einen Minimalwert und nicht mehr auf Null zurückgeht.

8. Verfahren nach einem der vorhergehenden Ansprüche, **dadurch gekennzeichnet, dass** nach Schritt c) eine Nachbehandlung des Implantatkörpers in einer wässrigen, stark basischen Natriumhydroxidlösung, vorzugsweise mit einem pH-Wert zwischen 11 und 13,8, zur Erzeugung einer Porengrundschicht erfolgt, wobei die Nachbehandlung unter Beaufschlagung von Ultraschall und/oder Einblasen von Argon und/oder Stickstoff durchgeführt wird.

9. Verfahren nach einem der vorhergehenden Ansprüche, **dadurch gekennzeichnet, dass** auf die plasmachemisch erzeugte Schicht eine weitere, vorzugsweise ein Polymer enthaltende Schicht (18), besonders bevorzugt enthaltend mindestens ein Polymer der Gruppe Parylene und PLA, insbesondere PLLA, aufgebracht wird.

10. Implantat, insbesondere intraluminale Endoprothese, mit einem Körper (12), wobei der Körper Magnesium oder eine Magnesiumlegierung aufweist, erhältlich durch ein Verfahren gemäß einem der vorhergehenden Ansprüche, wobei die metallischen Beschichtung (14) nach dem Schritt b) die erste Schichtdicke aufweist und eine zweite Schichtdicke der plasmachemisch erzeugten Schicht (16) nach Schritt c) größer ist als die erste Schichtdicke.

11. Implantat nach Anspruch 10, **dadurch gekennzeichnet, dass** die zweite Schichtdicke mindestens 0,3 µm, vorzugsweise mindestens 1 µm beträgt.

12. Implantat nach einem der Ansprüche 10 bis 11, **dadurch gekennzeichnet, dass** die plasmachemisch erzeugte Schicht (16) Poren aufweist.

13. Implantat nach einem der Ansprüche 10 bis 12, **dadurch gekennzeichnet, dass** die plasmachemisch erzeugte Schicht (16) mindestens eine Verbindung ausgewählt aus der Gruppe enthaltend
- Legierungen aus dem Material des Implantatkörpers und des Materials der metallischen Schicht, insbesondere Legierungen aus Magnesium und Titan, und
- Phosphate, Hydroxide und Oxide des Materials des Implantatkörpers und des Materials der metallischen Schicht, insbesondere Phosphate, Hydroxide und Oxide des Magnesiums und des Titans, aufweist.

## Claims

1. A method for producing an implant, in particular an intralumimal endoprosthesis, having a body (12), wherein the body comprises magnesium or a magnesium alloy, comprising the following steps:
a) preparing the implant body (12),
b) applying a metallic coating (14) onto at least a portion of the body surface, the primary constituent of the coating being titanium,
c) subjecting the portion of the body surface provided with the coating to plasmachemical treatment in an aqueous solution in order to produce a layer (16) created by plasmachemical treatment by applying a mixed voltage generating the plasma to the body of the implant, wherein the mixed voltage has sufficient energy to temporarily ionise both the metallic coating (14) and a subjacent region (15) of the implant body (12), and wherein the plasmachemical treatment of the body surface is carried out in that a pulsed voltage is applied to the body, the peak voltage of which in the predominant part of the treatment period exceeds at least 90 V and increases in the course of the treatment to 450 V and the energy lies in range of from 0.1 Ws to 10 Ws.

2. The method according to claim 1, **characterised in that** a first layer thickness of the metallic coating (14) after completing step b) is from approximately 0.1 µm to approximately 3 µm.

3. The method according to any one of the preceding claims, **characterised in that** the metallic coating (14) is applied by way of an ionic fluid, by way of a PVD method, or by way of a CVD method.

4. The method according to any one of the preceding claims, **characterised in that** the implant is rinsed in a solvent, preferably distilled H₂O, after step c) and is then dried, preferably at a temperature of at least about 80°C, particularly preferably at least 100°C, wherein the drying process is preferably carried out in a circulating air oven.

5. The method according to any one of the preceding claims, **characterised in that** the aqueous solution comprises Sr²⁺ ions, which are preferably present in the aqueous solution in a concentration of 0.05 mol/l to 2.0 mol/l Sr²⁺.

6. The method according to any one of the preceding claims, **characterised in that** the aqueous solution additionally comprises one or more ions selected from the group consisting of phosphates, carbonates and silicates, and/or that a buffer, preferably potassium dihydrogen phosphate and/or sodium dihydrogen phosphate, is present in the aqueous solution.

7. The method according to any one of the preceding claims, **characterised in that** the voltage in step c) after passing through the peak voltage returns to a minimal value and does not return to zero.

8. The method according to any one of the preceding claims, **characterised in that** after step c) an after-treatment of the implant body is carried out in an aqueous, strongly basic sodium hydroxide solution, which preferably has a pH value between approximately 11 and 13.8, in order to create a pore base layer, wherein the after-treatment is conducted by applying ultrasound and/or injecting argon and/or nitrogen.

9. The method according to any one of the preceding claims, **characterised in that** a further layer (18), preferably containing a polymer, particularly preferably containing at least one polymer selected from the group of parylene and PLA, in particular PLLA, is applied to the layer created by plasmachemical treatment.

10. An implant, in particular an intraluminal endoprosthesis, having a body (12), wherein the body comprises magnesium or a magnesium alloy, which can be obtained by a method according to any one of the preceding claims, wherein the metallic coating (14) after the step b) has the first layer thickness and a second layer thickness of the layer (16) created by plasmachemical treatment after step c) which is greater than the first layer thickness.

11. The implant according to claim 10, **characterised in that** the second layer thickness is at least 0.3 µm, preferably at least 1 µm.

12. The implant according to any one of claims 10 to 11, **characterised in that** the layer (16) created by plasmachemical treatment has pores.

13. The implant according to any one of claims 10 to 12, **characterised in that** the layer (16) created by plasmachemical treatment comprises at least one compound selected from the group containing
- alloys made of the material of the implant body and the material of the metallic layer, in particular alloys made of magnesium and titanium, and
- phosphates, hydroxides and oxides of the material of the implant body and of the material of the metallic layer, in particular phosphates, hydroxides and oxides of magnesium and titanium.

## Revendications

1. Procédé de fabrication d'un implant, notamment d'une endoprothèse intraluminale, avec un corps (12), où le corps présente du magnésium ou un alliage de magnésium, comprenant les étapes suivantes :
a) fourniture du corps d'implant (12),
b) application d'un revêtement (14) métallique, avec pour constituant principal le titane, sur au moins une partie de la surface de corps,
c) traitement chimique par plasma de la partie de la surface de corps munie du revêtement avec une solution aqueuse pour la fabrication d'une couche (16) créée de manière chimique par plasma au moyen de l'application d'une tension mixte générant le plasma sur le corps de l'implant, où la tension mixte présente une énergie suffisante pour qu'à la fois le revêtement (14) métallique et également la zone (15) se situant en dessous du corps d'implant (12) sont ionisés périodiquement et où le traitement chimique par plasma de la surface de corps se produit par le fait qu'une tension par impulsions est appliquée au corps, dont la tension de pic dépasse au moins 90 V dans la partie principale de la durée de traitement et augmente jusqu'à 450 V au cours du traitement et que l'énergie se situe dans la plage de 0,1 Ws à 10 Ws.

2. Procédé selon la revendication 1, **caractérisé en ce qu'**une première épaisseur de couche du revêtement (14) métallique est après l'achèvement de l'étape b) d'environ 0,1 µm à environ 3 µm.

3. Procédé selon l'une des revendications précédentes, **caractérisé en ce que** le revêtement (14) métallique est rapporté au moyen d'un liquide ionique, au moyen d'un procédé de dépôt physique en phase vapeur PVD ou au moyen d'un dépôt chimique en phase vapeur CVD.

4. Procédé selon l'une des revendications précédentes, **caractérisé en ce que** l'implant est lavé après l'étape c) dans un solvant, de préférence au moyen d'H₂O distillée et est ensuite séché de préférence à une température d'au moins 80 °C, de manière particulièrement préférée d'au moins 100 °C, où le séchage est de préférence exécuté dans un four à circulation d'air.

5. Procédé selon l'une des revendications précédentes, **caractérisé en ce que** la solution aqueuse contient des ions Sr²⁺ qui sont de préférence contenus dans une concentration de respectivement 0,05 Mol/l à 2,0 Mol/l de Sr²⁺ dans la solution aqueuse.

6. Procédé selon l'une des revendications précédentes, **caractérisé en ce que** la solution aqueuse présente en outre un ou plusieurs ions choisis dans le groupe des phosphates, des carbonates et des silicates et/ou que, dans la solution aqueuse, est contenu un tampon, de préférence, du dihydrogéno phosphate de potassium et/ou du dihydrogéno phosphate de sodium.

7. Procédé selon l'une des revendications précédentes, **caractérisé en ce que** la tension dans l'étape c) retourne à une valeur minimale et non plus à zéro après le passage de la tension de pic.

8. Procédé selon l'une des revendications précédentes, **caractérisé en ce qu'**après l'étape c), un nouveau traitement du corps d'implant a lieu dans une solution d'hydroxyde de sodium aqueuse, fortement basique, de préférence, avec une valeur de pH entre 11 et 13,8, pour la création d'une couche de base poreuse, où le nouveau traitement est exécuté moyennant la mise en oeuvre d'ultrasons et/ou d'un soufflage à l'argon et/ou à l'azote.

9. Procédé selon l'une des revendications précédentes, **caractérisé en ce que** sur la couche créée de manière chimique par plasma, une nouvelle couche (18) contenant de préférence un polymère, de manière particulièrement préférée, contenant au moins un polymère du groupe des parylènes et des acides polylactiques PLA, notamment de l'acide polylactique PLLA, est rapportée.

10. Implant, notamment endoprothèse intraluminale, avec un corps (12), où le corps présente du magnésium ou un alliage de magnésium, pouvant être obtenu par un procédé selon l'une des revendications précédentes, où le revêtement (14) métallique présente une première épaisseur de couche d'après l'étape b) et une deuxième épaisseur de couche de la couche (16) créée de manière chimique par plasma d'après l'étape c) est supérieure à la première épaisseur de couche.

11. Implant selon la revendication 10, **caractérisé en ce que** la deuxième épaisseur de couche est d'au moins 0,3 µm, de préférence d'au moins 1 µm.

12. Implant selon l'une des revendications 10 à 11, **caractérisé en ce que** la couche (16) créée de manière chimique par plasma présente des pores.

13. Implant selon l'une des revendications 10 à 12, **caractérisé en ce que** la couche (16) créée de manière chimique par plasma présente au moins un composé choisis dans le groupe contenant
- des alliages à base du matériau du corps d'implant et du matériau de la couche métallique, notamment des alliages à base de magnésium et de titane, et
- des phosphates, des hydroxydes et des oxydes du matériau du corps d'implant et du matériau de la couche métallique, notamment des phosphates, des hydroxydes et des oxydes du magnésium et du titane.
